# EUROPEAN PATENT SPECIFICATION

(11) **EP 0 310 187 B1**
(45) Date of publication and mention of the grant of the patent: **02.06.1993**
(21) Application number: 88202099.3
(22) Date of filing: 26.09.1988
(51) Int. Cl.: C07D 307/88

(54) **Process for preparing diphenyl ethers**
Verfahren zur Herstellung von Diphenylethern
Procédé pour préparer des éthers diphényliques

(30) Priority: 30.09.1987 GB 8722968
(43) Date of publication of application: 05.04.1989
(73) Proprietor: SHELL INTERNATIONALE RESEARCH MAATSCHAPPIJ B.V., 2596 HR Den Haag (NL)
(72) Inventor: Briggs, Stuart Peter, Faversham Kent (GB)

(56) References cited:
- EP-A- 0 145 078
- EP-A- 0 219 144
- EP-A- 0 265 020
- EP-A- 0 306 095
- EP-A- 0 306 096
- JP-A- 5 084 563
- US-A- 4 334 915
- Chemical Abstracts 95:80715m & US-A-4 298 530

## Description

This invention relates to a process for preparing diphenyl ether derivatives and the use of such derivatives as intermediates in the preparation of certain diphenyl ether herbicides.

Adaptations of the Knoevenagel-Doebner process for the selective preparation of substituted 3-hydroxy-3-methyl phthalides have been proposed in JP-50-84563 and US-A-4,298,530. The conditions proposed in JP-50-84563 lead to selective formation of 6-substituted phthalides, and US-A-4,298,530 proposes a complex variant of the conditions, requiring a solvent which is a dialkylformamide, a dialkylsulphoxide or an aliphatic lower carboxylic acid, and a metal salt catalyst with the aim of, inter alia, ensuring the same selective isomer formation when the method is carried out on an industrial scale.

The applicants' copending UK Patent Application No. 8720509 describes and claims herbicidal phenoxy phthalide derivatives having the general formula I: 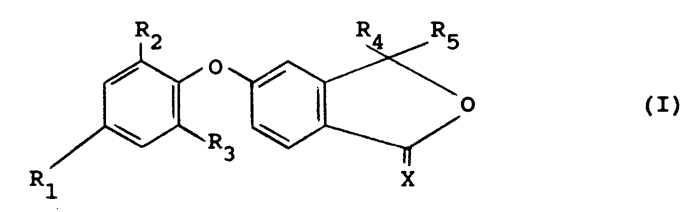
wherein R₁ represents a hydrogen or halogen atom, preferably chlorine, or an alkyl or haloalkyl group, suitably of 1-4 carbon atoms, preferably trifluoromethyl; R₂ and R₃, which may be the same or different, each independently represents a hydrogen or halogen atom, preferably chlorine, or an alkyl or haloalkyl group, suitably of 1-4 carbon atoms, for example trifluoromethyl, or a nitro or cyano group; R₄ represents a saturated alkyl group; R₅ represents an unsaturated alkyl group; and X represents an oxygen or sulphur atom.

The applicants' copending European Patent Application Publication No. 219144 similarly describes and claims herbicidal phenoxy phthalide derivatives having the formula (I) above where X is oxygen and R₄ and R₅, which may be the same or different, each independently represents an alkyl group.

The synthesis of such derivatives, especially where R₄ and R₅ are not identical, can be complex. Thus for example, in accordance with the above mentioned EP-A-21944, the synthesis of a compound when R₄ is methyl and R₅ is ethyl is carried out by first reacting the corresponding 3-hydroxy phthalide (ie. a compound of formula I where R₄ is hydrogen and R₅ is hydroxy) with a Grignard reagent (eg. methyl magnesium bromide) to give the 3-methyl phthalide and then reacting the product with an alkyl halide (eg. iodoethane) in the presence of lithium diisopropylamine to give the desired 3-methyl-3-ethyl phthalide.

According to the present invention we provide a process for the preparation of a diphenyl ether derivative of formula II 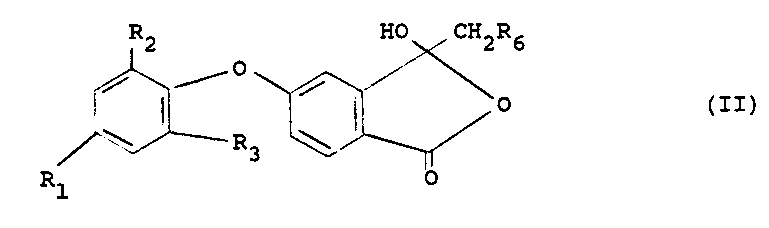
wherein R₁ represents a hydrogen or halogen atom, preferably chlorine, or an alkyl or haloalkyl group, suitably of 1-4 carbon atoms, preferably trifluoromethyl; R₂ and R₃, which may be the same or different, each independently represents a hydrogen or halogen atom, preferably chlorine, or an alkyl or haloalkyl group, suitably of 1-4 carbon atoms, for example trifluoromethyl, or a nitro or cyano group; and R₆ represents a hydrogen atom or an alkyl group, preferably having up to 4 carbon atoms, preferably methyl, comprising treating a compound of formula III 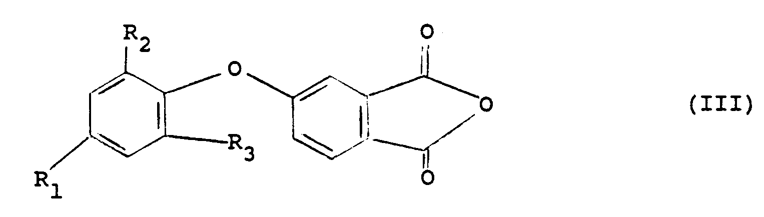
where R₁, R₂, and R₃ are as defined above with a dicarboxylic acid of formula R₆-CH(COOH)₂, where R₆ is as defined above, in the presence of an organic base.

The dicarboxylic acid is preferably malonic acid giving a compound of formula II where R₆ is hydrogen.

The organic base may be an aliphatic base such as triethylamine, an aromatic carboxylic base such as N,N-dimethyl aniline, or an aromatic heterocyclic base such as pyridine. The base preferably acts as solvent for the reaction.

The reactants are preferably employed using in excess of 1 mole equivalent of dicarboxylic acid per mole of compound of formula III, for example in a molar ratio of 1 to 2 mole equivalents of acid per mole of anhydride. The reaction temperature is preferably from room temperature to 100°C, or lower dependent on the decomposition point of the dicarboxylic acid employed. A preferred reaction temperature is from 70 to 90°C, preferably about 80°C with a reaction time of about 5 hours.

The reaction may be followed by any necessary separation procedures such as chromatography and/or recrystallisation from, for example, petrol/diethyl ether to remove unwanted isomeric by-products due to reaction with the second carbonyl group of the anhydride III. However in practice it has usually been found that the desired compound of formula II is obtained as the major product.

The anhydrides of formula III are known for example from US Patent Specification No. 4 334 915. They may be prepared for example by reaction of a compound of 3,4-dimethylphenol with a substituted halobenzene followed by oxidation of the methyl groups to acid groups and ring closure to form the anhydrides of formula III.

The invention also includes compounds of formula II when prepared by the process described above.

Compounds of formula II above are valuable intermediates for the preparation of compounds of formula I. Their use in the preparation of compounds of formula I where R₄ represents an alkyl group and R₅ represents an alkenyl or alkynyl group is described and claimed in our copending UK Patent Application No. 8720509. They are however also valuable in the preparation of compounds of formula I where R₄ and R₅, which may be the same or different, each independently represents an alkyl group.

Thus according to a further aspect of this invention we provide a process for the preparation of a compound of formula I where R₄ represents the group -CH₂R₆ and R₅ represents an alkyl, alkenyl or alkynyl group comprising reacting a compound of formula II which has been prepared as described above with an organometallic compound of formula R₅-M-Hal where M represents a metal atom and Hal represents a halogen atom, and optionally converting the compound of formula I where X represents an oxygen atom to a compound of formula I where X represents a sulphur atom.

The moiety Hal may be a chlorine, bromine or iodine atom.

The organometallic reagent used is preferably an organomagnesium compound (Grignard reagent), which may be prepared according to established procedures, e.g. by taking up the appropriate alkyl halide and magnesium metal in an aliphatic ether, such as diethyl ether in the absence of water. The reaction of the compound of formula II with that Grignard reagent is suitably carried out in a solvent, which may also be diethyl ether, or may be a different inert organic solvent such as tetrahydrofuran. The formation of the Grignard reagent and its reaction with the compound of formula II are each suitably carried out at ambient temperatures, although temperatures up to the boiling point of the solvent used may be employed. The Grignard organomagnesium complex may be supplemented by the generation of an organocadmium complex through the addition of cadmium chloride.

The invention also includes compounds of formula I when prepared by the process defined above.

Preferably in the compound of formula I, R₁ is trifluoromethyl, R₂ is a chlorine atom, R₃ is hydrogen, X is oxygen, R₄ is a methyl group and R₅ is an ethyl group, a vinyl group or an ethynyl group.

The invention is illustrated in the following Examples.

### Example 1

### 5-(2-chloro-4-trifluoromethylphenoxy)-phthalic anhydride

a) Sodium hydroxide (740g, 18.5mol), 3,4-dimethyl-phenol (2255g, 18.5mol) and sulpholane (7.51) were mixed and stirred at room temperature for 1 hour. Petroleum ether b.p. 100-120°C (21) was added and the mixture heated to reflux and water removed by azeotropic distillation. After 7 hours further sodium hydroxide (74g, 1.85mol) was added and refluxing continued until water ceased distilling over. 3-chloro-4-fluoro-benzotrifluoride (3672g, 18.5mol) was added at 80-90°C. After completion of the reaction and cooling, the reaction mixture was poured into cold water and the organic layer separated. After washing with sodium hydroxide and water the organic phase was concentrated to yield an oil (5178g.) distilling at 0.2mm Hg/120°C.
b) The product of step a) (300.5g, 1.0mol) in acetic acid (600ml) and acetic anhydride (26ml) was mixed with cobalt acetate (9g, 0.036mol) and cobalt bromide (6g, 0.018mol). The mixture was stirred, heated to 100°C and oxygen passed through at about 2 1.min⁻1. After the exothermic reaction subsided, solvent was removed by distillation and the residue triturated with toluene to give a white solid (239g) m.p. 170-170°C.
c) Product obtained as in step b (730g, 2.03mol) in xylene (2.5l) was heated under reflux and water removed by azeotropic distillation. After 6 hours reflux the mixture was cooled, filtered and xylene removed by distillation to give a yellow oil which crystallised from petroleum spirit.

Yield of the title compound 624g. m.p. 88-91°C.

### Example 2

### Preparation of 5-(2-chloro-4-trifluoromethyl-phenoxy)-3-hydroxy-3-methylphthalide

Dried malonic acid (156g, 1.5 mol) and 5-(2-chloro-4-trifluoromethylphenoxy)phthalic anhydride (342g, 1.0 mol) were stirred with triethylamine (200ml) under a nitrogen atmosphere and warmed slowly to 80°C. After 5 hours at 80°C, the reaction mixture was allowed to cool to room temperature and ethyl acetate (750ml) and water (500ml) were added. The organic phase was separated and washed with dilute hydrochloric acid then water. Ethyl acetate was distilled from the solution and the crude product purified by chromatography over silica gel. Finally, crystallisation from 40-60° petroleum spirit/diethyl ether afforded the product as a white solid, m.p. 106-108°C. Yield = 154g (43%).

| Analysis: | | |
|---|---|---|
| Calculated %: | C 53.58; | H 2.81 |
| Found %: | C 53.7; | H 2.8 |

### Example 3

### Preparation of 5-(2-chloro-4-trifluromethylphenoxy)-3-methyl-3-vinyl phthalide

Vinyl bromide (112.4g, 1.05 mol) in THF (500ml) was added to magnesium (24g, 1.0 mol) in THF (500ml) under a nitrogen blanket over 1 hour. The temperature was maintained below 60° during addition and stirring continued for a further 30 minutes during which all the magnesium dissolved. The reaction mixture was cooled to 0°C and then a solution of 5-(2-chloro-4-trifluoromethylphenoxy)-3-hydroxy-3-methyl-phthalide, prepared as described in Example 2, (119.5g, 0.33 mol) in THF (500ml) was run in while maintaining a temperature of 0 to 5°C. After stirring for a further 1 hour, during which the temperature rose to 20°C, dilute hydrochloric acid (1.1 mol in 250ml) was added very cautiously over 30 minutes. The solution separated into two layers and the aqueous layer was run off. Toluene (500ml) was added and the solution washed twice with water. The solvent was stripped and the residual oil dissolved in ethyl acetate/60-80° petroleum spirit and eluted over silica. The desired product, 5-(2-chloro-4-trifluoromethyl-phenoxy)-3-methyl-3-vinyl phthalide, was crystallised from hexane, m.p. = 71-73°C. Yield = 122.2g (96%).

| Analysis: | | |
|---|---|---|
| Calculated %: | C 58.63; | H 3.28 |
| Found %: | C 58.5; | H 3.3 |

### Example 4

### Preparation of 5-(2-chloro-4-trifluoromethyl-phenoxy)-3-ethyl-3-methylphthalide

Magnesium (1.44g, 0.06mol) suspended in diethyl ether (20ml), was treated with a crystal of iodine, then a solution of bromoethane (6.54g, 0.06mol) in diethyl ether (15ml) was added dropwise over 30 minutes. Reflux was maintained throughout this period by the heat of reaction. The mixture was stirred for 45 minutes before a solution of 5-(2-chloro-4-trifluoro-methylphenoxy)-3-hydroxy-3-methylphthalide (5.38g, 0.015mol) dissolved in diethyl ether (20ml) was added over 20 minutes. After stirring for a further 40 minutes, 50% hydrochloric acid (12ml) was cautiously added, then the phases separated. The organic phase was washed with water (3 × 15ml), the solvent stripped off and the residual oil distilled. Yield of pale yellow oil = 4.9g (88%).

| Analysis: | | |
|---|---|---|
| Found %: | C 58.7; | H 3.9 |
| Calculated %: | C 58.3; | H 3.9 |

### Example 5

### Preparation of 5-(2-chloro-4-trifluoromethylphenoxy)-3-ethyl-3-hydroxyphthalide

Dried 2-methylmalonic acid (2.07g, 0.0175 mol) and 5-(2-chloro-4- trifluoromethylphenoxy) phthalic anhydride (4g, 0.0117 mol) were stirred with triethylamine (2.36g, 0.0234 mol) and warmed slowly to 75°C. After 5 hours at 75°C the reaction mixture was allowed to cool to room temperature and ethyl acetate (50ml) and water (20ml) were added. The organic phase was separated, then washed with dilute hydrochloric acid and then water. Solvent was distilled from the organic solution and the residue was eluted over silica gel with a mixture of ethyl acetate/petroleum spirit. The desired product crystallised from hexane/diethyl ether. Yield = 2.0g (46%). mp 120-122.5°C.

## Claims

1. A process for the preparation of a diphenyl ether derivative of formula II 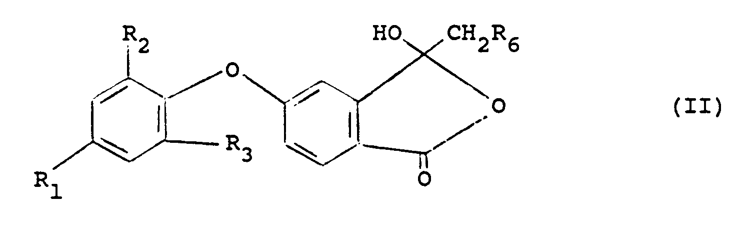 wherein R₁ represents a hydrogen or halogen atom or an alkyl or haloalkyl group, R₂ and R₃, which may be the same or different, each independently represents a hydrogen or halogen atom, or an alkyl, haloalkyl, nitro or cyano group, and R₆ represents a hydrogen atom or an alkyl group comprising treating a compound of formula III 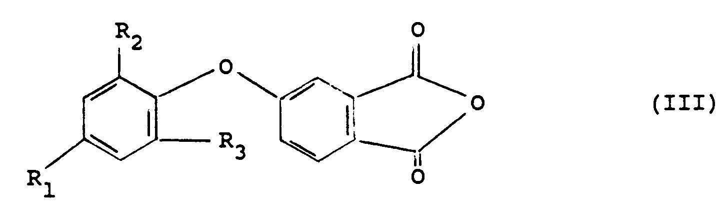 where R₁, R₂ and R₃ are as defined above with a dicarboxylic acid of formula R₆-CH(COOH)₂, where R₆ is as defined above, in the presence of an organic base.

2. A process according to claim 1, wherein R₁ is CF₃, R₂ is chlorine and R₃ is hydrogen.

3. A process according to claim 1 or 2, wherein R₆ is a hydrogen atom.

4. A process according to any one of the preceding claims, wherein the dicarboxylic acid is malonic acid.

5. A process according to any one of the preceding claims, wherein the organic base is triethylamine.

6. A process for the preparation of a compound of formula I 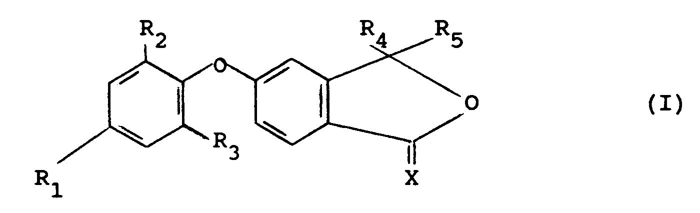 where R₁, R₂ and R₃ are as defined in claim 1, X represents an oxygen or sulphur atom, R₄ represents the group -CH₂R₆ where R₆ is as defined in claim 1 and R₅ represents an alkyl, alkenyl or alkynyl group, comprising reacting a compound of formula II which has been prepared according to any one of claims 1 to 5 with an organometallic compound of formula R₅-M-Hal where M represents a metal atom and Hal represents a halogen atom, and optionally converting the compound of formula I where X represents an oxygen atom to a compound of formula I where X represents a sulphur atom.

7. A process according to claim 6, wherein the organometallic compound is an organo-magnesium compound.

8. A process according to claim 6 or 7, wherein R₁ is trifluoromethyl, R₂ is a chlorine atom, R₃ is hydrogen, X is oxygen, R₄ is a methyl group and R₅ is an ethyl group, a vinyl group or an ethynyl group.

## Patentansprüche

1. Ein Verfahren zur Herstellung eines Diphenylätherderivats der Formel II 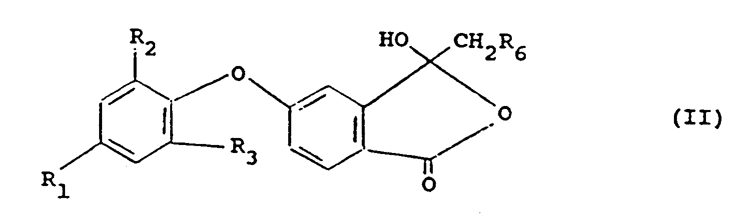 in welcher R₁ ein Wasserstoff- oder Halogenatom oder eine Alkyl- oder Halogenalkylgruppe ist, R₂ und R₃, welche gleich oder verschieden sein können, jeweils unabhängig ein Wasserstoff- oder Halogenatom, oder eine Alkyl-, Halogenalkyl-, Nitro- oder Cyanogruppe sind , und R₆ ein Wasserstoffatom oder eine Alkylgruppe ist, umfassend das Behandeln einer Verbindung der Formel III 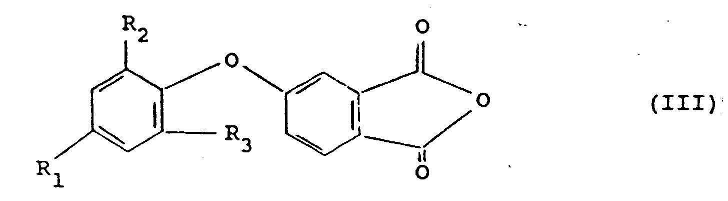 in welcher R₁, R₂ und R₃ wie oben definiert sind, mit einer Dicarbonsäure der Formel R₆-CH(COOH)₂, wobei R₆ wie oben definiert ist, in Gegenwart einer organischen Base.

2. Ein Verfahren nach Anspruch 1, in welchem R₁ CF₃ ist, R₂ Chlor ist und R₃ Wasserstoff bedeutet.

3. Ein Verfahren nach Anspruch 1 oder 2, in welchem R₆ ein Wasserstoffatom ist.

4. Ein Verfahren nach einem der vorstehenden Ansprüche, in welchem die Dicarbonsäure Malonsäure ist.

5. Ein Verfahren nach einem der vorstehenden Ansprüche, in welchem die organische Base Triethylamin ist.

6. Ein Verfahren nach einem der vorstehenden Ansprüche zur Herstellung einer Verbindung der Formel I 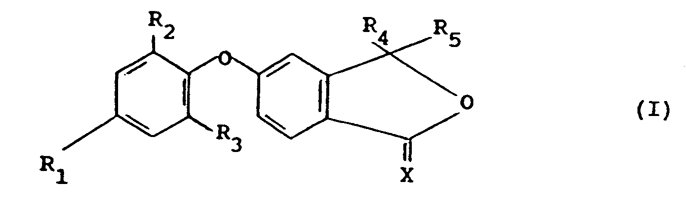 in welcher R₁, R₂ und R₃ wie in Anspruch 1 definiert sind, X ein Sauerstoff- oder Schwefelatom ist, R₄ die Gruppe -CH₂R₆ ist, wobei R₆ wie in Anspruch 1 definiert ist, und R₅ eine Alkyl-, Alkenyl- oder Alkynylgruppe ist, umfassend das Umsetzen einer Verbindung der Formel II , die nach einem der Ansprüche 1 bis 6 hergestellt worden ist, mit einer organometallischen Verbindung der Formel R₅-M-Hal, wobei M ein Metallatom ist und Hal ein Halogenatom darstellt, und gegebenenfalls die Umwandlung der Verbindung der Formel I, in welcher X ein Sauerstoffatom ist, in eine Verbindung der Formel I, in welcher X ein Schwefelatom bedeutet.

7. Ein Verfahren nach Anspruch 6, in welchem die organometallische Verbindung eine Organo-Magnesiumverbindung ist.

8. Ein Verfahren nach Anspruch 6 oder 7, in welchem R₁ Trifluormethyl ist, R₂ ein Chloratom ist, R₃ Wasserstoff ist, X Sauerstoff ist , und R₄ eine Methylgruppe ist und R₅ eine Ethylgruppe, eine Vinylgruppe oder eine Ethynylgruppe ist.

## Revendications

1. Un procédé pour la préparation d'un dérivé d'éther diphénylique de formule II: 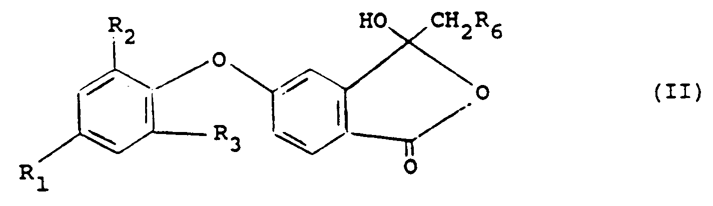 dans laquelle R₁ représente un atome d'hydrogène ou d'halogène ou bien un groupe alkyle ou haloalkyle, R₂ et R₃, qui peuvent être identiques ou différents, représentent chacun, de façon indépendante, un atome d'hydrogène ou d'halogène ou bien un groupe alkyle ou haloalkyle, nitro ou cyano; et R₆ représente un atome d'hydrogène ou un groupe alkyle, qui consiste à traiter un composé de formule III: 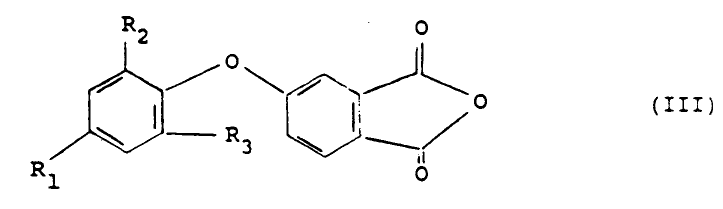 dans laquelle R₁, R₂ et R₃ sont tels que définis ci-dessus, avec un acide dicarboxylique de formule R₆-CH(COOH)₂, dans laquelle R₆ est tel que défini ci-dessus, en présence d'une base organique.

2. Un procédé selon la revendication 1, dans lequel R-1 est CF₃, R₂ est du chlore et R₃ est de l'hydrogène.

3. Un procédé selon la revendication 1 ou 2, dans lequel R₆ est un atome d'hydrogène.

4. Un procédé selon l'une quelconque des revendications précédentes, dans lequel l'acide dicarboxylique est l'acide malonique.

5. Un procédé selon l'une quelconque des revendications précédentes, dans lequel la base organique est la triéthylamine.

6. Un procédé selon l'une quelconque des revendications précédentes, pour la préparation d'un composé de formule I 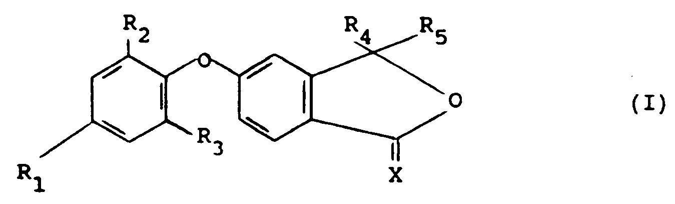 dans laquelle R₁, R₂ et R₃ sont telles que définies dans la revendication 1, X représente un atome d'oxygène ou de soufre, R₄ représente le groupe -CH₂R₆ dans lequel R₆ est tel que défini dans la revendication 1 et R₅ représente un groupe alkyle, alcényle ou alcynyle, procédé dans lequel on fait réagir un composé de formule II qui a été préparé selon l'une quelconque des revendications 1 à 6 avec un composé organo-métallique de formule R₅-M-Hal dans lequel M représente un atome de métal et Hal représente un atome d'halogène, et, facultativement, on convertit le composé de formule I dans lequel X représente un atome d'oxygène en un composé de formule I dans laquelle X représente un atome de soufre.

7. Un procédé selon la revendication 6, dans lequel le composé organo-métallique est un composé organo-magnésium.

8. Un procédé selon la revendication 6 ou 7, dans lequel R₁ est du trifluorométhyle, R₂ est un atome de chlore, R₃ est de l'hydrogène, X est de l'oxygène, R₄ est un groupe méthyle et R₅ est un groupe éthyle, un groupe vinyle ou un groupe éthynyle.
